# EUROPEAN PATENT APPLICATION

(11) **EP 4 755 407 A2**
(43) Date of publication of application: **10.06.2026**
(21) Application number: 26159151.5
(22) Date of filing: 19.08.2020
(51) Int. Cl.: A61L 27/36

(54) **SEALING OF DECELLULARIZED AND RECELLULARIZED ENGINEERED ORGAN GRAFTS**

(30) Priority: 19.08.2019 US 201962888703 P
(62) Divisional of application: 20764535.9
(71) Applicant: Miromatrix Medical Inc., Eden Prairie, Minnesota 55344 (US)
(72) Inventor: KATANE, Aleksandr, Victoria, Minnesota, 55386 (US); ROSS, Jeffrey, Chaska, Minnesota, 55318 (US); DAVIDOW, Dominique, Minnetonka, Minnesota, 55345 (US); RIESGRAF, Shawn, Carver, Minnesota, 55315 (US)
(74) Representative: Murgitroyd & Company

(57) **Abstract**

The disclosure provides for methods of sealing of decellularized and recellularized engineered organ grafts, thereby providing a reinforced (fortified) decellularized and recellularized engineered organ graft.

## Description

### Cross-Reference to Related Applications

This application claims the benefit of the filing date of U.S. application No. 62/888,703, filed on August 19, 2019, the disclosure of which is incorporated by reference herein.

### Background

The major types of connective tissue are connective tissue proper, supportive tissue, and fluid tissue. Loose connective tissue proper includes adipose tissue, areolar tissue, and reticular tissue. These serve to hold organs and other tissues in place and, in the case of adipose tissue, isolate and store energy reserves. The matrix is the most abundant feature for loose tissue although adipose tissue does not have much extracellular matrix. Dense connective tissue proper is richer in fibers and may be regular, with fibers oriented in parallel as in ligaments and tendons, or irregular, with fibers oriented in several directions. Organ capsules (collagenous type) contain dense irregular connective tissue. Dense connective tissue is reinforced by bundles of fibers that provide tensile strength, elasticity, and protection. When surgeons are faced with a compromised donor organ surface during transplant they are able to remedy the bleeding by applying commercially available bioglues, fibrin sealants, hemostatic sponges and other solutions e.g., Surgifoam or Tisseel. Moreover, bleeding from liver injuries, whether they occur due to trauma before or during surgery may be treated with gelatin gelfoams, oxidized cellulose, microfibrillar collagen, thrombin, thrombin with gelatin or fibrin sealant.

### Summary

This disclosure provides for decellularized bio-engineered organs or tissues, or portions thereof, that are modified, e.g., coated so as to provide a seal, so as to have a fortified (reinforced) outer layer (the outer fibrous layer comprising the outer extracellular matrix which has a composition that, in one embodiment, is at least 50% non-fibrous collagen in contrast to the parenchyma which has more fibrillar collagen than non-fibrous collagen) with properties, such as strength, elongation, elasticity, abrasion resistance, modulus of elasticity, and/or porosity properties, that are similar to or enhanced relative to native organ or tissue outer fibrous layers. In one embodiment, the modified outer layer has increased strength, increased elongation, increased elasticity, increased abrasion resistance, increased modulus of elasticity, and/or decreased porosity properties relative to a corresponding native organ or tissue outer fibrous layer. This disclosure also provides for recellularized bio-engineered organs or tissues, or portions thereof, that are modified to have a fortified outer layer with properties that are similar to or enhanced relative to native organ or tissue outer fibrous layers. The decellularized or recellularized bio-engineered organ, tissue, or portion thereof, is contacted with a biocompatible composition, e.g., an aqueous liquid, a powder or a gel composition, that has one or more agents that, when coated on the surface of the organ, tissue, or portion thereof, seals the outer surface and, if present, any edges, e.g., of the portions, so that an aqueous liquid that is exogenously introduced and/or circulated in the vasculature of the organ, tissue or portion thereof, is retained in the organ, tissue or portion to a greater extent, e.g., at least 5%, 10% or more retention, than when the aqueous liquid is introduced and/or circulated in the vasculature of a corresponding organ, tissue or portion thereof, that is not contacted with the composition. In one embodiment, pressure is used to compare retention of the liquid, e.g., at about or above physiological pressures and optionally at corresponding flows or at double or triple the pressure, e.g., 5 to 10 mmHg, 10 to 22 mmHg, 20 to 40 mmHg, 50 to 60 mmHg, 60 to 100 mmHg, 100 to 150 mmHg or more, where the modified outer (exterior) surface inhibits fluid transfer across the outer surface to a greater extent than the unmodified outer surface. Thus, the pressures at which the liquid is applied can be at physiological pressures or at greater pressures including but not limited to a 5%, 7%, 10%, 20%, 50%, or 100% increase or more in native pressure. For example, if physiological pressures in native liver are about 8 to 12 mmHg, liquid introduced to a liver having a fortified (reinforced) outer layer which is subjected to pressures of at least 8 to 12 mmHg, e.g., at least 14 to 18 mmHg, would be retained in that liver to a greater extent than a corresponding liver without a fortified outer layer. For portal delivery of liquid to the liver, physiological pressure is about 6 to about 10 mm Hg. For delivery of liquid to the lung, physiological pressure is about 12 to 14 mmHg. For arterial flow, physiological pressure is about 80 to 120 mmHg and for venous supply, physiological pressure is about 8 to 14 mmHg. Thus, a fortified outer layer of an organ will have enhanced retention of liquid at pressures that are at least physiological pressures but also at greater than physiological pressures relative to a corresponding unfortified outer layer of the organ. In one embodiment, there is less beading of an introduced aqueous liquid on a dry exterior surface of an organ after contact with the composition relative to dry exterior surface of an organ that is not contacted with the composition. In one embodiment, there is less escape (volume) of an introduced aqueous liquid, e.g., over time, from an organ after contact with the composition relative to an organ that is not contacted with the composition. In one embodiment, the composition is a gel and the agent therein allows for crosslinking of molecules on the outer (exterior) surface directly or indirectly to each other. In one embodiment, the crosslinking occurs as a result of a change in pH or temperature. In one embodiment, the agent is a crosslinker which is activated by light, e.g., UV light, is employed to initiate crosslinking. In one embodiment, the composition comprises a polymer, e.g., a biocompatible synthetic or natural polymer including proteins, and that polymer is crosslinked to molecules on the outer surface of the organ, tissue or portion thereof, and optionally to other polymer molecules. In one embodiment, the polymer comprises collagen, e.g., mammalian collagen such as human or porcine collagen, including but not limited to porcine dermis collagen, or gelatin derived therefrom, and optionally not bovine collagen. The collagen may be of any molecular weight, e.g., from 100 to 10,000 Da, 10,000 to 100,000 Da, or 100 kDa to 500. In one embodiment, the composition comprises an enzyme, e.g., a transferase, for instance, a transglutaminase, a hydrolase, for instance, a peptidase, an oxidoreductase, such as lysyl oxidase, tyrosinase, laccase, or peroxidase, or a hydroxylase such as prolyl hydroxylase. In one embodiment, the composition may include agent that reduces adhesion formation once the modified (coated) organ, tissue or portion thereof is implanted. In one embodiment, an agent that reduces adhesion formation, e.g., laminin or collagen IV, may be applied after the organ, tissue or portion thereof, is coated with the composition. In one embodiment, the agent that reduces adhesion formation provides for a smooth surface, e.g., the agent comprises polypropylene. In one embodiment the composition comprises of a hydrophilic polymer. In one embodiment the composition comprises a hydrophobic polymer. In one embodiment, the modification alters the mechanical properties of the outer fibrous layer which may be measured for example, by ball burst, tensile strength, tear test, and/or liquid leak.

Thus, a method to decrease porosity (e.g., efflux of fluid from) of an outer fibrous layer of a decellularized mammalian organ or tissue, or a portion thereof, is provided. The method can enable the development of tissues, organs or portions of that can also sustain greater pressures without fluid loss through the outer organ surface. The method includes providing a decellularized extracellular matrix of the mammalian organ or tissue, or portion thereof, which comprises an exterior surface comprising decellularized extracellular matrix and comprises a decellularized extracellular matrix vascular tree, wherein said decellularized extracellular matrix vascular tree of said organ or tissue, or portion thereof, retains a majority but not all of fluid introduced thereto. At least the exterior surface is exposed to or contacted with *ex vivo* a composition comprising at least one agent in an amount that forms a biocompatible coating on the exterior surface of the decellularized extracellular matrix of the mammalian organ or tissue, or portion thereof, effective to increase retention of fluid introduced to the decellularized organ, tissue or portion thereof.

### Brief Description of the Figures

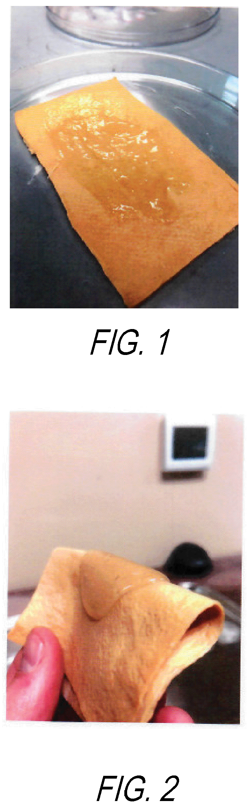

Fig. 1 demonstrates the deposit of a gelatin-based substance that has been crosslinked to the surface of liver extracellular matrix in order to seal and reinforce the surface. The substance is able cover the surface area uniformly.
Fig. 2 demonstrates that the crosslinked substance is able to stretch and bend on the surface of the extracellular matrix. This demonstrates that the fortifying substance has the mechanical properties to be handled by operators during culturing, transport, implantation and therapy.

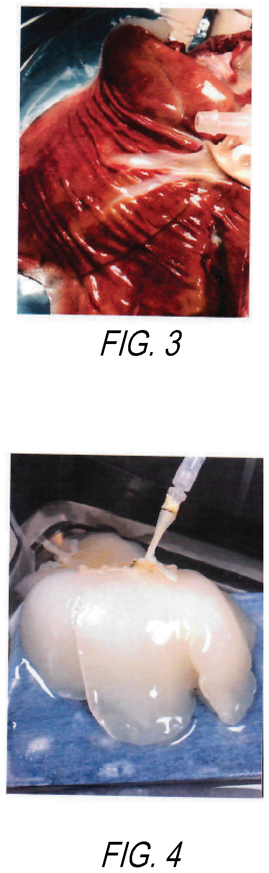

Fig. 3 demonstrates the deposit of an exemplary reinforcing/fortifying composition on the surface of a harvested porcine liver. The whole surface of the liver is covered with a thin layer of the composition in order to seal and strengthen the capsule. The figure demonstrates that the composition adhered to the surface of a liver after crosslinking and is able to bend and stretch with the surface of the outer capsule.
Fig. 4 demonstrates a decellularized liver graft that is perfused with DI water at about 350 ml/min with leaking through the surface of the capsule. This is evident with the beading of DI water on the surface of the capsule.

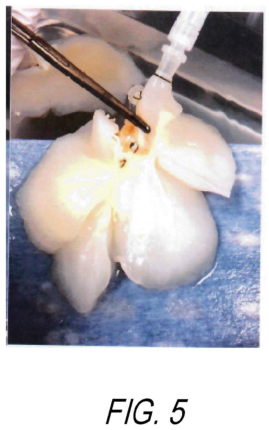

Fig. 5 demonstrates a decellularized liver graft that has had the surface treated and fortified/sealed with a gelatin substance that is UV crosslinked to the surface of the graft. It is being perfused with DI water at about 350 ml/min but there is no leaking of the liquid through the outer surface.

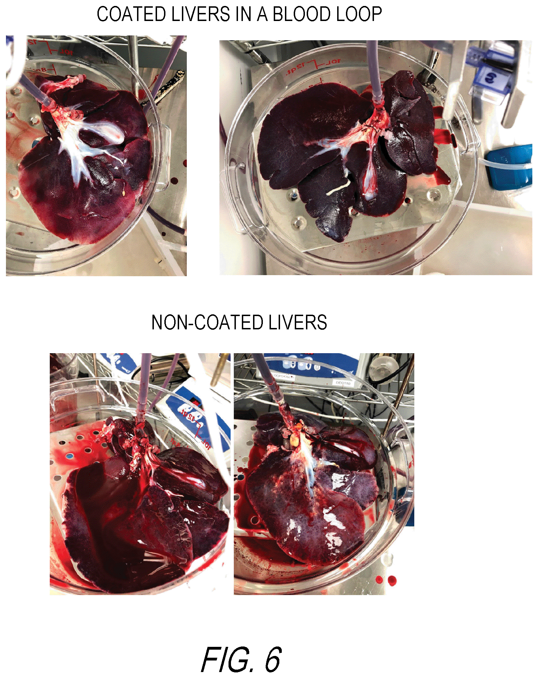

Fig. 6. Polymer coated and non-coated recellularized porcine livers (coated, decellularized and recellularized with human endothelial cells) were subjected to an *ex vivo* blood loop at a constant flow of 350ml/min, consistent with native portal flow. During the *ex vivo* blood loop liver grafts remained patent with a glucose consumption rate >80 mg/hr. Coated (with a benzophenone based polymer that allows for cross-linking when exposed to UV light) grafts reduced the amount of fluid transfer through the outer capsule compared to non-coated grafts. These livers were decellularized and revascularized with human endothelial cells to enable a patency assay and demonstration of the effective coating of organs.

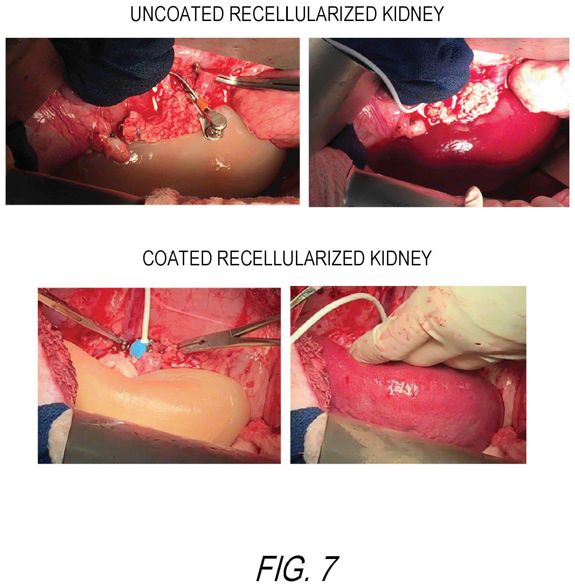

Fig. 7. Polymer coated and non-coated recellularized porcine kidneys (decellularized, coated and recellularized) were implanted into a pig via direct anastomosis of the renal artery and renal vein. Following reperfusion, the coated grafts reduced the amount of fluid transfer through the outer surface compared to non-coated kidneys.

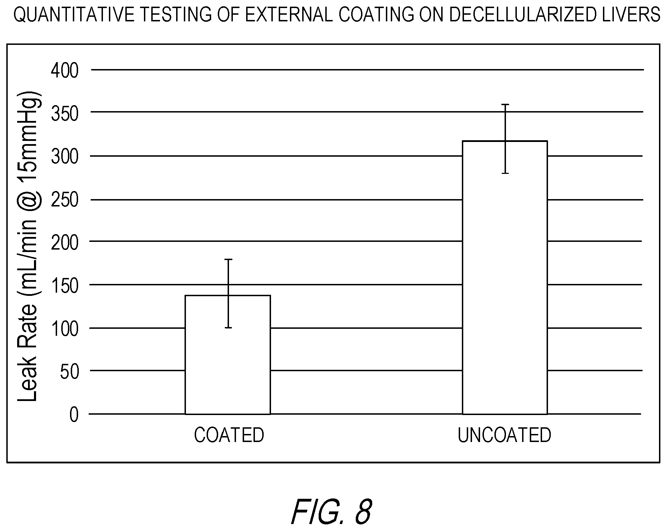

Fig. 8. Average leak rate (mL/min at 15mmHg) of decellularized coated and non-coated porcine liver grafts. Leak rates are calculated at a pressure of 15mmHg with capped vessels and standard PBS solution that has a lower viscosity than blood. Coated decellularized livers demonstrated a significant reduction in their leak rate compared to non-coated decellularized grafts. A polymer coating was applied to freshly isolated livers prior to decellularization. The coating was robust and lasted through the decellularization process adhering to the external surface.

### Detailed Description

The boundary and outer surface (in some cases this is referred to as a capsule) of an organ or tissue serve an important role. That role may be compromised after decellularization. For example, during the decellularization process the integrity of an organ's boundary or surface decreases, e.g., due to the removal of cells near the outer surface or the lack of internal cells which reduces the bulk properties of the organ or tissue. This may result in a decellularized organ or tissue graft that is prone to percolate (leak) liquid when perfused, has reduced mechanical strength or flexibility, is prone to have fissures, or has diminished handling properties, e.g., so that it is easy to puncture the unmodified decellularized organ or tissue. In order to be physiologically compatible, an engineered organ graft should have an outer surface that is able to retain the inner flow of blood in the vasculature without leaking to the outside. Thus, although the outer surface of a decellularized organ remains macroscopically intact or substantially intact, particularly if subjected to perfusion decellularization, the removal of the outermost cellular layer may result in at least some leakage to the outside if fluid is introduced to the decellularized vasculature. The present disclosure provides for an outer surface of an engineered organ or tissue graft that has resilient handling and installation properties, e.g., which are conducive to successful implantation. This can be accomplished by fortifying and/or sealing the outside of the graft by, in one embodiment, depositing an additive layer of chemistry or cross-linking molecules on the cell surface or molecules added to the cell surface, e.g., via a linker, that can strengthen the graft, e.g., crosslink the outer layer of the extracellular matrix. Thus, the disclosure provides for methods of reinforcement, fortification and/or sealing of decellularized and recellularized engineered organ grafts.

In one embodiment, the fortification of the outside of a bioengineered organ graft may be accomplished by depositing a substance (compound(s)) that can seal the outside of the decellularized graft. This substance can be in the form of a powder, a liquid, or gel. The substance that is deposited may allow for crosslinking. The substance may be applied to the entire outer (exterior) surface, or a portion thereof that is suspected of not having integrity. Once applied to the organ, tissue or portion thereof, the deposited layer of substance, with or without further reactions, acts as a physical barrier, thus sealing the organ, tissue or portion thereof, against leaks and/or strengthening the organ, tissue or portion thereof, e.g., liver lobe, heart valve, lung lobe, left ventricle, right ventricle, kidney pole, etc. for improved handling properties. Once applied, the organ, tissue or portion thereof, is robust enough to have a range of bending and stretching in order for the organ, tissue or portion thereof to accommodate the graft/organ shape, e.g., once recellularized. The deposited layer stays adhered to the surface of the graft, e.g., during subsequent handling including, for instance, throughout the culturing/manufacturing period, transportation period and/or after implantation. The compound (agent) that is applied and the resulting layer that is formed are biocompatible and so can be implanted and not cause adverse effects. This can include a modification that would prevent, inhibit or reduce adhesion formations following transplantation. The substance that is applied may also contain molecules that prevent the formation of adhesions on the surface of the graft.

In one embodiment, the deposited substance can be a gelatin or a gelatin-like substance, e.g., one that is matched to the species of organ to which it is to be applied, that is initially flowable but then sets and adheres once it is deposited on the surface of the graft. Flowable substances may include, but are not limited to, gelatin, agar or carrageenan. In one embodiment, the substance may have properties that enable it to adhere to the surface of the graft by crosslinking with the collagen on the surface of the graft. Additive catalytic crosslinking agents may include, but are not be limited to, transglutaminase.

Other additives include but are not limited to polymers, e.g., polyvinylpyrrolidone, photopolyvinylpyrrolidone, PEG, antibodies, amino acids or proteins, e.g. Type I Collagen, Type 2 Collagen, Type 3 Collagen, Laminin, RGD, Type 4 collagen, and the like.

The flowable deposited substance may contain one or more photoreactive crosslinkers and/or a secondary component crosslinks onto the surface of the graft, e.g., by UV exposure. In one embodiment, the photoreactive crosslinkers, with UV exposure, can induce a covalent bond between solution components and the collagen surface of the graft. This enables the components of the solution to adhere to the surface of the graft and fortify and seal the graft surface.

In one embodiment, a sprayable deposited substance may contain one or more photoreactive crosslinkers and/or a secondary component that crosslinks onto the surface of the organ or tissue graft, e.g., by UV exposure. The curation on the crosslinker can be pH, chemical, acoustic, heat, light, water, or laser initiated. The substance may be sprayed out of a spray nozzle or a powered spray head. This enables a uniform application of the substance onto the surface of the graft.

An organ graft may be submerged in a composition contains one or more photoreactive crosslinkers and/or a secondary component that crosslinks onto the surface of the graft. The composition may also contain an enzyme, e.g., a transferase, for instance, a transglutaminase, a hydrolase, for instance, a peptidase, an oxidoreductase, such as lysyl oxidase, tyrosinase, laccase, or peroxidase, or a hydroxylase such as prolyl hydroxylase. The graft may be submerged in the composition and subjected to UV exposure for surface curing to enable crosslinking. The pH of the composition may be altered to activate the enzymatic action to crosslink the surface of the graft while it is submerged in the composition.

A powder substance having one or more enzymes or photo cross-linkable polymers may be deployed onto the surface of a graft. The presence of moisture on the surface of the graft enables the powder to go into solution and allows for flowable liquid covering on the surface. The surface pH can trigger enzymatic action to crosslink the surface of the graft. Once the graft surface is covered it may be subject to UV radiation to crosslink the polymers with each other and the graft surface.

### General Methods of Making Decellularized and Recellularized Organs

A solid organ refers to an organ that has a "substantially closed" vasculature system. A "substantially closed" vasculature system with respect to an organ means that, upon perfusion with a liquid, the majority of the liquid is contained within the solid organ and does not leak out of the solid organ, assuming the major vessels are cannulated, ligated, or otherwise restricted. Despite having a "substantially closed" vasculature system, many of the organs listed above have defined "entrance" and "exit" vessels which are useful for introducing and moving the liquid throughout the organ during perfusion. In addition, other types of vascularized organs or tissues such as, for example, all or portions of joints (e.g., knees, shoulders, or hips), anus, trachea, or spinal cord, can be perfusion decellularized. Further, avascular tissues such as, for example, cartilage or cornea, may be decellularized when part of a larger vascularized structures such as a whole leg.

Perfusion decellularization of tissue or organ ECM provides an intact ECM that has the ability to provide the structural, biochemical, and mechanical properties, e.g., to enable functional cell differentiation and maintenance. Although perfusion decellularization of organ or tissue ECM is superior to immersion in terms of preserving an intact matrix with structural and biochemical cues, including intact vasculature, the compositions described herein are useful for both perfusion and immersion decellularized organs, tissues or portions thereof, e.g., greater than 2 cm³ and/or a thickness that exceeds about 2 mm in thickness.

Perfusion decellularized matrices of organs with a substantially closed vascular system are particularly useful because perfusion decellularization preserves the intact matrix and microenvironment, including an intact vascular and microvascular system, that vascular system may be utilized to deliver cells as well as nutrients and/or differentiation or maintenance factors, to the cells *in vitro.* Cells and nutrients and/or other factors may be delivered by other means, e.g., injection, or passive means, or a combination thereof. In one embodiment, a cell population of interest is perfused into the perfusion decellularized organ ECM allowing for the seeding into the interstitial space or matrix outside of the vascular conduits. This includes the active migration and/or homing of cells to their native microstructure, e.g. the homing of beta cells to islet structures within the pancreas matrix. In one embodiment, a cell population of interest is perfused into the perfusion decellularized ECM followed by a second cell population, e.g., beta cell population, followed by an endothelial cell population, where the endothelial cells remain in the vascular conduits as in their native microenvironment. In another embodiment, two or more cell populations are combined and perfused together. In another embodiment, two or more distinct cell populations are introduced serially through either perfusion, direct injection or a combination of both. For example, hepatocytes may be introduced to a perfusion decellularized liver matrix either through perfusion or injection followed by the seeding of endothelial cells. In another embodiment, perfusion decellularized liver matrix is seeded with fetal liver cells including endothelial and epithelial cells. In another embodiment, fetal liver cells are seeded in the matrix followed by endothelial cells. In one embodiment, fetal lung cells containing a mixture of all lung cell types including epithelial, endothelial and interstitial lineages, are seeded through perfusion into the lung vasculature and airway of a perfusion decellularized lung, for instance, to create a functional lung construct such as one that provides for further maturation of the isolated lung cells. In one embodiment, a perfusion decellularized heart from a small mammal, e.g., a rat, rabbit, mouse, guinea pig or ferret, is perfused with partially differentiated human cardiomyocytes, human cardiac fibroblasts, human smooth muscle cells, and human endothelial cells to create a beating miniaturized human heart, e.g., for pharmaceutical testing.

The cells may be introduced in media that support the proliferation, metabolism, and/or differentiation of the cells. Alternatively, after the cells have populated the ECM, the medium is changed to one that supports the proliferation, metabolism and differentiation of the cells. The cultured cells may exist in the ECM at physiological cell densities and, in the presence of media that support the proliferation, metabolism, and/or differentiation of the cells and/or the appropriate microenvironment in the ECM, allow for the maintenance and/or functional differentiation of the cells.

### Decellularization of Organs or Tissues

Decellularization generally includes the following steps: stabilization of the solid organ, e.g., a vascuarlized structure thereof, or tissue, decellularization of the solid organ or tissue, renaturation and/or neutralization of the solid organ or tissue, washing the solid organ, degradation of any DNA remaining on the organ, disinfection of the organ or tissue and homeostasis of the organ.

The initial step in decellularizing an organ vascularized structure or tissue is to cannulate the organ or tissue. The vessels, ducts, and/or cavities of an organ or tissue tissue may be cannulated using methods and materials known in the art. Next, the cannulated organ vascuarlized structure or tissue is perfused with a cellular disruption medium. Perfusion through an organ can be multidirectional (e.g., antegrade and retrograde).

Langendorff perfusion of a heart is routine in the art, as is physiological perfusion (also known as four chamber working mode perfusion). See, for example, Dehnert, The Isolated Perfused Warm-Blooded Heart According to Langendorff, In Methods in Experimental Physiology and Pharmacology: Biological Measurement Techniques V. Biomesstechnik-Verlag March GmbH, West Germany, 1988.

Briefly, for Langendorff perfusion, the aorta is cannulated and attached to a reservoir containing physiological solution to allow the heart to function outside of the body for a specified duration of time. To achieve perfusion decellularization the protocol has been modified to perfuse a cellular disruption medium delivered in a retrograde direction down the aorta either at a constant flow rate delivered, for example, by an infusion or roller pump or by a constant hydrostatic pressure pump. In both instances, the aortic valves are forced shut and the perfusion fluid is directed into the coronary ostia (thereby perfusing, via antegrade, the entire ventricular mass of the heart), which then drains into the right atrium via the coronary sinus. For working mode perfusion, a second cannula is connected to the left atrium and perfusion can be changed to retrograde.

In one embodiment, a physiological solution includes phosphate buffer saline (PBS). In one embodiment, the physiological solution is a physiologically compatible buffer supplemented with, e.g., nutritional supplements (for instance, glucose). For example, for heart, the physiological solution may be Modified Krebs-Henseleit buffer having 118 mM NaCl, 4.7 mM KC1, 1.2 mM MgSO₄, 1.2 mM KH₂PO₄, 25 mM NaHCO₃, 11 mM glucose, 1.75 mM CaCl₂, 2.0 mM pyruvate and 5 U/L insulin; or Krebs buffer containing 118 mM NaCl, 4.7 mM KC1, 25 mM NaHCO₃, 1.2 mM MgSO₄, 1.2 mM KH₂PO₄, 2 mM CaCl₂ gassed with 95% O₂, 5% CO₂. Hearts may be perfused with glucose (e.g., about 11 mM) as a sole substrate or in combination with about 1 or 1.2 mM palmitate. For kidney, the physiological solution may be KPS-1^{®} Kidney Perfusion Solution. For liver, the
physiological solution may be
Krebs-Henseleit buffer having 118 mM NaCl, 4.7 mM KC1, 1.2 mM MgSO₄, 1.2 mM KH₂PO₄, 26 mM NaHCO₃, 8 mM glucose, and 1.25 mM CaCl₂ supplemented with 2% BSA.

Methods are known in the art for perfusing other organ or tissues. By way of example, the following references describe the perfusion of lung, liver, kidney, brain, and limbs. Van Putte et al., Ann. Thorac. Surg., 74(3):893 (2002); den Butter et al., Transpl. Int., 8:466 (1995); Firth et al., Clin. Sci. (Lond.), 77(6):657 (1989); Mazzetti et al., Brain Res., 999(1):81 (2004); Wagner et al., J. Artif. Organs, 6(3):183 (2003).

One or more cellular disruption media may be used to decellularize an organ or tissue. A cellular disruption medium generally includes at least one detergent such as but not limited to SDS, PEG, CHAPS or Triton X. A cellular disruption medium can include water such that the medium is osmotically incompatible with the cells. Alternatively, a cellular disruption medium can include a buffer (e.g., PBS) for osmotic compatibility with the cells. Cellular disruption media also may include enzymes such as, without limitation, one or more collagenases, one or more dispases, one or more DNases, or a protease such as trypsin. In some instances, cellular disruption media also or alternatively may include inhibitors of one or more enzymes (e.g., protease inhibitors, nuclease inhibitors, and/or collegenase inhibitors).

In certain embodiments, a cannulated organ or tissue may be perfused sequentially with two different cellular disruption media. For example, the first cellular disruption medium may include an anionic detergent such as SDS and the second cellular disruption medium can include an ionic detergent such as Triton X. Following perfusion with at least one cellular disruption medium, a cannulated organ or tissue may be perfused, for example, with wash solutions and/or solutions containing one or more enzymes such as those disclosed herein. Alternating the direction of perfusion (e.g., antegrade and retrograde) may assist in decellularizing the entire organ or tissue. Decellularization generally decellularizes the organ from the inside out, resulting in very little damage to the ECM. An organ or tissue may be decellularized at a suitable temperature between 4 and 40°C. Depending upon the size and weight of an organ or tissue and the particular detergent(s) and concentration of detergent(s) in the cellular disruption medium, an organ or tissue generally is perfused from about 0.05 hours to about 5 hours, per gram of solid organ or tissue (generally > 50 grams), or about 2 hours to about 12 hours, per gram of solid organ or tissue for organs (generally < 50 grams), with cellular disruption medium. Including washes, an organ may be perfused for up to about 0.75 hours to about 10 hours per gram of solid organ or tissue (generally >50 grams), or about 12 hours to about 72 hours, per gram of tissue (generally <50 grams). Decellularization time is dependent upon the vascular and cellular density of the organ or tissue with limited scaling for overall mass. Therefore, as general guidance the time ranges and masses above are provided. Perfusion generally is adjusted to physiologic conditions including pulsatile flow, rate and pressure.

A decellularized organ or tissue has the extracellular matrix (ECM) component of all or most regions of the organ or tissue, including ECM components of the vascular tree. ECM components can include any or all of the following: fibronectin, fibrillin, laminin, elastin, members of the collagen family (e.g., collagen I, III, and IV), ECM associated growth proteins including growth factors and cytokines, glycosaminoglycans, ground substance, reticular fibers and thrombospondin, which can remain organized as defined structures such as the basal lamina. Successful decellularization is defined as the absence of detectable myofilaments, endothelial cells, smooth muscle cells, and nuclei in histologic sections using standard histological staining procedures or removal of over 97% of detectable DNA as measured by fluorometric assay. Residual cell debris may be removed from the decellularized organ or tissue.

The morphology and the architecture of the ECM is maintained during and following the process of decellularization. "Morphology" as used herein refers to the overall shape of the organ, tissue or of the ECM, while "architecture" as used herein refers to the exterior surface, the interior surface, and the ECM therebetween.

The morphology and architecture of the ECM may be examined visually and/or histologically. For example, the basal lamina on the exterior surface of a solid organ or within the vasculature of an organ or tissue should not be removed or significantly damaged due to perfusion decellularization. In addition, the fibrils of the ECM should be similar to or significantly unchanged from that of an organ or tissue that has not been decellularized.

One or more compounds may be applied in or on a decellularized organ or tissue to, for example, preserve the decellularized organ, or to prepare the decellularized organ or tissue for recellularization and/or to assist or stimulate cells during the recellularization process. Such compounds include, but are not limited to, one or more growth factors (e.g., VEGF, DKK-1, FGF, BMP-1, BMP-4, SDF-1, IGF, and HGF), immune modulating agents (e.g., cytokines, glucocorticoids, IL2R antagonist, leucotriene antagonists), and/or factors that modify the coagulation cascade (e.g., aspirin, heparin-binding proteins, and heparin). In addition, a decellularized organ or tissue may be further treated with, for example, irradiation (e.g., UV, gamma) to reduce or eliminate the presence of any type of microorganism remaining on or in a decellularized organ or tissue.

### Recellularization of Organs or Tissues

A decellularized organ or tissue is contacted with a population of cells, either differentiated (mature or primary) cells, stem cells, or partially differentiated cells. Thus, the cells can be totipotent cells, pluripotent cells, or multipotent cells, and can be uncommitted or committed, and may be single-lineage cells. The cells may be undifferentiated cells, partially differentiated cells, or fully differentiated cells including fetal derived cells. Cells may include progenitor cells, precursor cells, or "adult" derived stem cells including umbilical cord cells and fetal stem cells. Cells useful in the matrices of the invention include embryonic stem cells (as defined by the National Institute of Health (NIH); see, for example, the Glossary at stemcells.nih.gov on the World Wide Web) and iPS cells.

Examples of cells that can be used to recellularize an organ or tissue include, without limitation, embryonic stem cells, umbilical cord blood cells, tissue-derived stem or progenitor cells, bone marrow-derived step or progenitor cells, blood-derived stem or progenitor cells, mesenchymal stem cells (MSC), skeletal muscle-derived cells, multipotent adult progentitor cells (MAPC), or iPS cells Additional cells that can be used include cardiac stem cells (CSC), multipotent adult cardiac-derived stem cells, cardiac fibroblasts, cardiac microvasculature endothelial cells, aortic endothelial cells, coronary endothelial cells, microvascular endothelial cells, venous endothelial cells, arterial endothelial cells, smooth muscle cells, cardiomyocytes, hepatocytes, beta-cells, keratinocytes, purkinji fibers, neurons, bile duct epithelial call, islet cells, pneumocytes, clara cells, brush boarder cells, or podocytes. Bone marrow-derived stem cells such as bone marrow mononuclear cells (BM-MNC), endothelial or vascular stem or progenitor cells, and peripheral blood-derived stem cells such as endothelial progenitor cells (EPC) may also be used as cells.

The number of cells that are introduced into and onto a perfusion decellularized scaffold may depend both the organ (e.g., which organ, the size and weight of the organ) or tissue and the type and developmental stage of the regenerative cells. Different types of cells may have different tendencies as to the population density those cells will reach. Similarly, different organ or tissues may be cellularized at different densities. By way of example, a decellularized organ or tissue can be "seeded" with at least about 1,000 (e.g., at least 10,000, 100,000, 1,000,000, 10,000,000, or 100,000,000) cells; or can have from about 1,000 cells/mg tissue (wet weight, e.g., prior to decellularization) to about 10,000,000 cells/mg tissue (wet weight) attached thereto.

Cells can be introduced ("seeded") into a decellularized organ or tissue by injection into one or more locations. In addition, more than one type of cell may be introduced into a decellularized organ or tissue. For example, a population of differentiated cell types can be injected at multiple positions in a decellularized organ or tissue or different cell types may be injected into different portions of a decellularized organ or tissue. Alternatively, or in addition to injection, cells or a cocktail of cells may be introduced by perfusion into a cannulated decellularized organ or tissue. For example, cells can be perfused into a decellularized organ using a perfusion medium, which can then be changed to an expansion and/or differentiation medium to induce growth and/or differentiation of the cells. Location specific differentiation may be achieved by placing cells into the various locations within the organ, e.g., into regions of the heart, such as, atrial, ventricular or nodal.

During recellularization, an organ or tissue is maintained under conditions in which at least some of the cells can multiply and/or differentiate within and on the decellularized organ or tissue. Those conditions include, without limitation, the appropriate temperature and/or pressure, electrical and/or mechanical activity, force, the appropriate amounts of O₂ and/or CO₂, an appropriate amount of humidity, and sterile or near-sterile conditions. During recellularization, the decellularized organ or tissue and the regenerative cells attached thereto are maintained in a suitable environment. For example, the cells may require a nutritional supplement (e.g., nutrients and/or a carbon source such as glucose), exogenous hormones or growth factors, and/or a particular pH.

Cells may be allogeneic to a decellularized organ or tissue (e.g., a human decellularized organ or tissue seeded with human cells), or cells may be xenogeneic to a decellularized organ or tissue (e.g., a pig decellularized organ or tissue seeded with human cells). "Allogeneic" as used herein refers to cells obtained from the same species as that from which the organ or tissue originated (e.g., related or unrelated individuals), while "xenogeneic" as used herein refers to cells obtained from a species different than that from which the organ or tissue originated.

Stem or progenitor media may contain a variety of components including, for example, KODMEM medium (Knockout Dulbecco's Modified Eagle's Medium), DMEM, Ham's F12 medium, FBS (fetal bovine serum), FGF2 (fibroblast growth factor 2), KSR or hLIF (human leukemia inhibitory factor). The cell differentiation media may also contain supplements such as L-Glutamine, NEAA (non-essential amino acids), P/S (penicillin/streptomycin), N2, B27 and beta-mercaptoethanol. It is contemplated that additional factors may be added to the cell differentiationmedia, including, but not limited to, fibronectin, laminin, heparin, heparin sulfate, retinoic acid, members of the epidermal growth factor family (EGFs), members of the fibroblast growth factor family (FGFs) including FGF2, FGF7, FGF8, and/or FGF10, members of the platelet derived growth factor family (PDGFs), transforming growth factor (TGF)/bone morphogenetic protein (BMP)/growth and differentiation factor (GDF) factor family antagonists including but not limited to noggin, follistatin, chordin, gremlin, cerberus/DAN family proteins, ventropin, high dose activin, and amnionless or variants or functional fragments thereof. TGF/BMP/GDF antagonists could also be added in the form of TGF/BMP/GDF receptor-Fc chimeras. Other factors that may be added include molecules that can activate or inactivate signaling through Notch receptor family, including but not limited to proteins of the Delta-like and Jagged families as well as inhibitors of Notch processing or cleavage, or variants or functional fragments thereof. Other growth factors may include members of the insulin like growth factor family (IGF), insulin, the wingless related (WNT) factor family, and the hedgehog factor family or variants or functional fragments thereof. Additional factors may be added to promote mesendoderm stem/progenitor, endoderm stem/progenitor, mesoderm stem/progenitor, or definitive endoderm stem/progenitor proliferation and survival as well as survival and differentiation of derivatives of these progenitors.

In one embodiment, perfusion decellularized matrices are combined with iPS or ES cells differentiated using the embryoid body (EB) method. For example, human iPS cell lines reprogrammed by transduction, e.g., lentiviral-mediated transduction, of transcription factors (*OCT4, SOX2, NANOG* and *LIN28*; Oct3/4, Sox2, Klf4, and c-Myc; or Oct3/4, Sox2, and Klf4) are employed. iPS clones of fetal origin or of newborn origin may be employed. Human ES cell lines may also be employed. iPS cells and ES cells may be maintained on irradiated mouse embryonic fibroblasts (MEFs) at a density of 19,500 cells/cm² in 6-well culture plates (Nunc) in DMEM/F12 culture medium supplemented with 20% KnockOut serum replacer (Invitrogen), 0.1 mmol/L nonessential amino acids, 1 mmol/L L-glutamine, and 0.1 mmol/L β-mercaptoethanol (Sigma). In addition, the medium may be supplemented with 100 ng/mL, zebrafish basic fibroblast growth factor for iPS cells, and with 4 ng/mL human recombinant basic fibroblast growth factor (Invitrogen) for hES cells. iPS and ES cell lines may also be maintained on gelatinized 100-mm dishes in DMEM (Sigma-Aldrich) containing 15% fetal calf serum (FCS; Sigma-Aldrich), 0.1 µmol/L 2-mercaptoethanol (2ME), and 1,000 units/ml LIF (Chemicon International). For differentiation, these cells may treated with 0.25% Trypsin/ethylenediaminetetraacetic acid (GIBCO), and transferred to gelatinized 6-well plates in α-minimum essential medium (GIBCO) supplemented with 10% FCS and 0.05 µmol/L 2ME, at a concentration of 3 × 10⁴ cells/well.

Colonies may be detached from culture plates by incubating with 1 mg/mL dispase (Gibco) solution at 37°C for 8 to 15 minutes and placed in ultralow attachment plates in suspension culture, e.g., for 4 days. During suspension culture, the medium may be changed at day 1 followed by culture for another 3 days without medium change. EBs are then plated on 0.1% gelatin-coated culture plates, e.g., at the density or 50 to 100 EBs per well, or in the perfusion decellularized ECM and cultured in differentiation medium (e.g., changed daily).

In some instances, an organ or tissue generated by the methods described herein is to be transplanted into a patient. In those cases, the cells used to recellularize a decellularized organ or tissue can be obtained from the patient such that the cells are "autologous" to the patient. Cells from a patient can be obtained from, for example, blood, bone marrow, tissues, or organs at different stages of life (e.g., prenatally, neonatally or perinatally, during adolescence, or as an adult) using methods known in the art. Alternatively, cells used to recellularize a decellularized organ or tissue may be syngeneic (i.e., from an identical twin) to the patient, cells can be human lymphocyte antigen (HLA)-matched cells from, for example, a relative of the patient or an HLA-matched individual unrelated to the patient, or cells can be allogeneic to the patient from, for example, a non-HLA-matched donor.

Irrespective of the source of the cells (e.g., autologous or not), the decellularized solid organ can be autologous, allogeneic or xenogeneic to a patient.

The progress of cells can be monitored during recellularization. For example, the number of cells on or in an organ or tissue can be evaluated by taking a biopsy at one or more time points during recellularization. In addition, the amount of differentiation that cells have undergone can be monitored by determining whether or not various markers are present in a cell or a population of cells. Markers associated with different cells types and different stages of differentiation for those cell types are known in the art, and can be readily detected using antibodies and standard immunoassays. See, for example, Current Protocols in Immunology, 2005, Coligan et al., Eds., John Wiley & Sons, Chapters 3 and 11. Nucleic acid assays as well as morphological and/or histological evaluation can be used to monitor recellularization.

### Exemplary Embodiments

In one embodiment, reinforcement of the organ surface may be accomplished prior to the decellularization process. The reinforcing substance may be deposited on to the surface of the harvested organ in order to reinforce and seal the outside of the organ. After deposition of the reinforcing substance, the organ is subjected to decellularization process during which the outside of the decellularized organ remains reinforced.

In one embodiment, reinforcement of the organ surface may be accomplished after the decellularization process and before recellularization. The reinforcing substance may be deposited on to the surface of the decellularized organ in order to reinforce and seal the outside of the decellularized graft. After depositing the reinforcement substance, the graft can be seeded and cultured while the surface remains reinforced.

In one embodiment, reinforcement of the organ surface may be accomplished after the recellularization process. The reinforcing substance may be deposited on to the surface of the recellularized graft to reinforce and seal the outside of the recellularized graft. After depositing the reinforcement substance, the graft can be cultured while the surface remains reinforced.

In one embodiment, reinforcement of the organ surface may be accomplished prior to implantation of the graft for therapy. The reinforcing substance may be deposited on to the surface of the recellularized graft to reinforce and seal the outside of the recellularized graft after transportation but prior to implantation. The recellularized bio-engineered organ may be surface treated with the reinforcing substance at the site of implantation.

In one embodiment, reinforcement of the organ surface may be accomplished prior to decellularization of the graft and optionally also after recellularization, e.g., prior to implantation.

In one embodiment, reinforcement of the organ surface may be accomplished prior to decellularization of the graft and optionally also prior to recellularization.

In one embodiment, a flowable reinforcing substance may be deployed onto the graft using methods such as, but not limited to, spraying, brushing/swabbing or otherwise coating the organ, e.g., by dipping (immersing) the organ into the substance.

In one embodiment, the reinforcing substance may be adhered to the graft surface by crosslinking the substance with surface of the graft, e.g., with the collagen on the surface of the graft. Crosslinking may be accomplished with, but not limited to, a catalyzing agent such as transglutaminase and other catalysts. Photo-induced crosslinking may be aided with the addition of photocrosslinkers in order to create bonds between the surface of the graft and additives. Photocrosslinkers may be used to crosslink the existing surface of the organ. Additives include, but are not limited to, polymers, amino acids and specific proteins.

Thus, the compositions disclosed herein may coat the entire surface of the graft. The coating may be applied prior any bleeding or oozing (leakage). The composition, besides creating a barrier, may have hemostatic properties, or may be non-thrombogenic. The coating may reduce the surface porosity through actions such as cross linking, or it may create an adhesive barrier coating with a reduced porosity in the outer coating.

Polymer hydrogels may be formed by using a bifunctional monomer or crosslinking macromolecules, optionally along with a photoinitiator or crosslinker. In one embodiment, a coating was formed using polyvinylpyrrolidone (PVP) applied to the exterior surface of an organ and UV light. In one embodiment, a coating was formed using polyethylene glycol (PEG) applied to the exterior surface of an organ and UV light. In one embodiment, a coating was formed using agar applied to the exterior surface of an organ and UV light. In one embodiment, a coating was formed using gelatin applied to the exterior surface of an organ and UV light. In one embodiment, a coating was formed using collagen applied to the exterior surface of an organ and UV light.

In one embodiment, the composition to be applied is a powder, e.g., collagen
powder with added catalyzers like transferases, hydrolases, or oxidoreductases that would activate once on the surface and crosslink the surface to the added collagen powder. For example, a 5% (by weight) porcine dermis gelatin is prepared by heating water to 45°C and adding the gelatin powder while the solution is stirring. 5% (by weight) Activa GS (transglutaminase) is added to the solution while it is stirring and at 45°C. The pH is then titrated to 6.5-7 by adding 1N Hydrochloric Acid. Once the pH is in the range, it is applied to the surface of the graft with a swab or by pouring. As the solution cools it will phase change into a gel state and then begin to crosslink due to the pH shift. Setting time ranges from 1 to 4hrs as the solution cools, sets and crosslinks onto the graft.

In one embodiment, the composition to be applied is a liquid solution, e.g., a liquid solution comprising a photoreactive crosslinking agent (such as photoactive polyvinylpyrrolidone, photopolyvinylpyrrolidone, benzophonoes, and the like) that is activated with UV light exposure and crosslinks to the surface of the collagen containing graft. In one embodiment, the composition to be applied is a gel, e.g., a liquid solution that contains gelatin-like components (such as gelatin, agar, carrageenan, and the like) that phase change into a gel once applied onto the surface of the graft. The phase change enables the coating to stay on to the graft and in one embodiment the result of a temperature, pH change, acoustic, chemical or water.

The resulting fortified organ has similar properties or improved properties relative to a native organ. For example, the organs having an applied coating may have mechanical properties, e.g., mechanical strength, that are similar to or improved relative to a native organ, e.g., measured using mechanical tests such as ball burst (ASTM D6797 - 15), pant tear (ASTM D1938 - 19) or tensile strength (ASTM D5034 - 09). The organs having an applied coating may have structural or hemodynamic properties that are similar to or improved relative to a native organ, e.g., as measured by a leak test or fluid transfer through the outer surface under a defined flow which could be physiological.

In one embodiment, the composition to be applied may be a polymer, e.g., a photoreactive polymer, that has hydrophobic properties, thereby providing for a hydrophobic surface that inhibits adhesions. In one embodiment, the composition to be applied is hydrophilic and may contain a substance that provides for a hydrophilic coating that is non adhesive. In one embodiment, the composition to be applied may modified proteins such as laminin or basement membrane substrates such as Collagen IV that enhance the non-adhesive properties of the coating.

In one embodiment, the non-adhesive component may be added after the coating is applied.

In one embodiment, an *ex vivo* method to decrease porosity of an outer fibrous layer of a decellularized mammalian organ or tissue, or a portion thereof is provided. The method includes providing a decellularized extracellular matrix of the mammalian organ or tissue, or portion thereof, e.g., > which is >8cm³, which comprises an exterior surface comprising decellularized extracellular matrix and comprises a decellularized extracellular matrix vascular tree, wherein said decellularized extracellular matrix of said organ or tissue, or portion thereof, retains a majority but not all of fluid introduced thereto; and exposing the exterior surface to a composition comprising at least one agent in an amount that forms a biocompatible coating on the exterior surface of the decellularized extracellular matrix of the mammalian organ or tissue, or portion thereof, effective to increase retention of fluid introduced to the decellularized extracellular matrix vascular tree relative to a corresponding decellularized extracellular matrix of the mammalian organ or tissue, or portion thereof, comprising an exterior surface comprising decellularized extracellular matrix and a decellularized extracellular matrix vascular tree which is not exposed to the composition. In one embodiment, the mammal is a swine, canine, feline, equine, caprine, bovine, ovine or human. In one embodiment, the organ is a liver, heart, spleen, pancreas, bladder, kidney, small bowel, large bowel, stomach, bone, brain, or a lung. In one embodiment, the composition comprises a powder, a gel or a liquid, e.g., aqueous liquid. In one embodiment, the amount of the composition enhances mechanical flexibility or strength of the decellularized extracellular matrix of the mammalian organ or tissue. In one embodiment, the method also includes introducing a population of cells to a duct, cavity or one or more vessels of the decellularized organ or tissue. In one embodiment, the cells are introduced before the exterior surface is exposed to the composition. In one embodiment, the cells are introduced after the exterior surface is exposed to the composition. In one embodiment, the composition comprises an agent that reduces the formation of adhesions. In one embodiment, the method further includes contacting the exterior surface with a composition comprising an agent that reduces the formation of adhesions. In one embodiment, the agent comprises polypropylene. In one embodiment, the agent comprises laminin, basement membrane substrates or collagen IV. In one embodiment, the composition is sprayed onto the exterior surface. In one embodiment, the organ or tissue is submerged in the composition. In one embodiment, the exposing results in crosslinking of molecules on the exterior surface. In one embodiment, the agent is an enzyme, e.g., a transferase, hydrolase, oxidoreductase or transglutaminase. In one embodiment, the molecule that is crosslinked comprises collagen. In one embodiment, the composition comprises a photocrosslinker. In one embodiment, the exterior surface is exposed to the composition and to a cross-linking agent. In one embodiment, the photocrosslinker comprises a polymer. In one embodiment, the composition comprises polyvinylpyrrolidone or photopolyvinylpyrrolidone. In one embodiment, the composition comprises poly(ethylene), poly(ethylene oxide), poly(vinyl alcohol), copolymers of maleic anhydride and/or phthalic anhydride with styrene and some polymers derived from cinnamic acid. In one embodiment, the composition comprises benzophenone. In one embodiment, the composition comprises one or more amino acids. In one embodiment, the composition comprises Type I Collagen, Type 2 Collagen, Type 3 Collagen, Laminin, RGD, or Type 4 collagen. In one embodiment, the agent comprises a bifunctional crosslinker. In one embodiment, the composition comprises gelatin, agar or carrageenan. In one embodiment, the composition comprises collagen. Further provided is a product produced by the method.

In one embodiment, retention of fluid in the coated decellularized organ, tissue, or portion thereof, is increased by at least 5%, 10%, 20%, 30%, 40% 50%, 70% or more relative to a corresponding uncoated decellularized organ, tissue, or portion thereof.

In one embodiment, the coated decellularized organ, tissue, or portion thereof, when liquid is introduced at physiological pressures or up to twice physiological pressures, has a loss of fluid of less than 15 ml/min, 12 ml/min, 10 ml/min, 7 ml/min, 5 ml/min, 2 ml/min or relative to a corresponding uncoated decellularized organ, tissue, or portion thereof, e.g., which may lose fluid at a rate that is greater than, e.g., 15 ml/min.

In one embodiment, an *ex vivo* method to decrease porosity of an exterior surface of a mammalian organ or tissue, or a portion thereof, is provided. The method includes providing a mammalian organ or tissue, or a portion thereof, wherein said organ or tissue, or portion thereof, comprises an exterior surface, a vascular tree and cells; and exposing the exterior surface of the organ or tissue, or portion thereof, to a composition comprising at least one agent in an amount that forms a coating on the exterior surface effective to increase retention of fluid in the organ or tissue relative to fluid introduced to a corresponding organ or tissue, or a portion thereof, prior to exposure to the composition. In one embodiment, the mammalian organ or tissue, or a portion thereof, has not been decellularized and recellularized. In one embodiment, the mammalian organ or tissue, or a portion thereof has been decellularized and recellularized. A product produced by the method is also provided.

In one embodiment, retention of fluid in the coated organ, tissue, or portion thereof, is increased by at least 5%, 10%, 20%, 30%, 40% 50%, 70% or more relative to a corresponding uncoated organ, tissue, or portion thereof.

In one embodiment, the coated organ, tissue, or portion thereof, when liquid is introduced at physiological pressures or up to twice physiological pressures, has a loss of fluid of less than 15 ml/min, 12 ml/min, 10 ml/min, 7 ml/min, 5 ml/min, 2 ml/min or 0 ml/min relative to a corresponding uncoated organ, tissue, or portion thereof, e.g., which may lose fluid at a rate that is greater than, e.g., 15 ml/min.

Thus, the methods include coating prior to decellularization, after decellularization or before implantation, or any combination thereof.

All publications, patents and patent applications are incorporated herein by reference. While in the foregoing specification, this invention has been described in relation to certain preferred embodiments thereof, and many details have been set forth for purposes of illustration, it will be apparent to those skilled in the art that the invention is susceptible to additional embodiments and that certain of the details herein may be varied considerably without departing from the basic principles of the invention.

The following is a list of non-limiting embodiment of the present invention:
Embodiment 1. A method to decrease porosity of an outer fibrous layer of a decellularized mammalian organ or tissue, or a portion thereof, comprising:
   providing a decellularized extracellular matrix of the mammalian organ or tissue, or portion thereof, which comprises an exterior surface comprising decellularized extracellular matrix and comprises a decellularized extracellular matrix vascular tree, wherein said decellularized extracellular matrix of said organ or tissue, or portion thereof, retains a majority but not all of fluid introduced thereto; and
   contacting the exterior surface to a composition comprising at least one agent in an amount that forms a biocompatible coating on the exterior surface of the decellularized extracellular matrix of the mammalian organ or tissue, or portion thereof, effective to increase retention of fluid introduced to the decellularized extracellular matrix vascular tree relative to a corresponding decellularized extracellular matrix of the mammalian organ or tissue, or portion thereof, comprising an exterior surface comprising decellularized extracellular matrix and a decellularized extracellular matrix vascular tree which is not contacted with the composition.
Embodiment 2. The method of Embodiment 1 wherein the mammal is a swine, bovine, ovine or human.
Embodiment 3. The method of Embodiment 1 or 2 wherein the organ is a liver, heart, spleen, pancreas, bladder, kidney, small bowel, large bowel, stomach, bone, brain, or a lung.
Embodiment 4. The method of any one of Embodiments 1 to 3 wherein the composition comprises a powder, a gel or a liquid.
Embodiment 5. The method of any one of Embodiments 1 to 4 wherein the amount of the composition enhances mechanical flexibility or strength of the decellularized extracellular matrix of the mammalian organ or tissue.
Embodiment 6. The method of any one of Embodiments 1 to 5 further comprising introducing a population of cells to a duct, cavity or one or more vessels of the decellularized organ or tissue.
Embodiment 7. The method of Embodiment 6 wherein the cells are introduced before the exterior surface is contacted with the composition.
Embodiment 8. The method of Embodiment 6 wherein the cells are introduced after the exterior surface is contacted with the composition.
Embodiment 9. The method of any one of Embodiments 1 to 8 wherein the composition comprises an agent that reduces the formation of adhesions.
Embodiment 10. The method of any one of Embodiments 1 to 8 further comprising contacting the exterior surface with a composition comprising an agent that reduces the formation of adhesions.
Embodiment 11. The method of Embodiment 9 or 10 wherein the agent comprises polypropylene.
Embodiment 12. The method of Embodiment 9 or 10 wherein the agent comprises laminin, basement membrane substrates or collagen IV.
Embodiment 13. The method of any one of Embodiments 1 to 12 wherein the composition is sprayed onto, brushed onto or applied to the exterior surface.
Embodiment 14. The method of any one of Embodiments 1 to 12 wherein the organ or tissue is submerged in the composition.
Embodiment 15. The method of any one of Embodiments 1 to 14 wherein the contacting results in crosslinking of molecules on the exterior surface.
Embodiment 16. The method of any one of Embodiments 1 to 15 wherein the agent is an enzyme.
Embodiment 17. The method of Embodiment 16 wherein the enzyme comprises a transferase, hydrolase or oxidoreductase.
Embodiment 18. The method of Embodiment 16 wherein the enzyme comprises transglutaminase.
Embodiment 19. The method of Embodiment 15 wherein the molecule on the exterior surface that is crosslinked to the at least one agent comprises collagen.
Embodiment 20. The method of any one of Embodiments 1 to 19 wherein the composition comprises a photocrosslinker.
Embodiment 21. The method of any one of Embodiments 1 to 19 wherein the exterior surface is further contacted with a photocrosslinker.
Embodiment 22. The method of Embodiment 20 or 21 wherein the photocrosslinker comprises a polymer.
Embodiment 23. The method of Embodiment 20 or 21 wherein the exterior surface is contacted with a polyvinylpyrrolidone or photopolyvinylpyrrolidone.
Embodiment 24. The method of Embodiment 20 or 21 wherein the exterior surface is contacted with a poly(ethylene), poly(ethylene oxide), poly(vinyl alcohol), copolymers of maleic anhydride and/or phthalic anhydride with styrene and some polymers derived from cinnamic acid.
Embodiment 25. The method of Embodiment 20 or 21 wherein the exterior surface is contacted with comprises benzophenone.
Embodiment 26. The method of any one of Embodiments 20 to 25 wherein the composition comprises one or more amino acids.
Embodiment 27. The method of any one of Embodiments 1 to 25 wherein the composition comprises Type I Collagen, Type 2 Collagen, Type 3 Collagen, Laminin, RGD, or Type 4 collagen.
Embodiment 28. The method of any one of Embodiments 1 to 27 wherein the at least one agent comprises a bifunctional crosslinker.
Embodiment 29. The method of any one of Embodiments 1 to 28 wherein the composition comprises gelatin, agar or carrageenan.
Embodiment 30. The method of any one of Embodiments 1 to 29 wherein the composition comprises collagen.
Embodiment 31. A product produced by the method of any one of Embodiments 1 to 30.
Embodiment 32. A method to decrease porosity of an exterior surface of a mammalian organ or tissue, or a portion thereof, comprising:
   providing a mammalian organ or tissue, or a portion thereof, wherein said organ or tissue, or portion thereof, comprises an exterior surface, a vascular tree and cells; and
   contacting the exterior surface of the organ or tissue, or portion thereof, with a composition comprising at least one agent in an amount that forms a coating on the exterior surface effective to increase retention of fluid in the organ or tissue relative to fluid introduced to a corresponding organ or tissue, or a portion thereof, prior to exposure to the composition.
Embodiment 33. The method of Embodiment 32 wherein the mammalian organ or tissue, or a portion thereof, has not been decellularized and recellularized.
Embodiment 34. The method of Embodiment 32 wherein the mammalian organ or tissue, or a portion thereof has been decellularized and recellularized. Embodiment 35. The method of Embodiment 32, 33 or 34 wherein the mammal is a swine, bovine, ovine or human.
Embodiment 36. The method of any one of Embodiments 32 to 35 wherein the organ is a liver, heart, spleen, pancreas, bladder, kidney, small bowel, large bowel, stomach, bone, brain, or a lung.
Embodiment 37. The method of any one of Embodiments 32 to 36 wherein the composition comprises a powder, a gel or a liquid.
Embodiment 38. The method of any one of Embodiments 32 to 37 wherein the amount of the composition enhances mechanical flexibility or strength of the decellularized extracellular matrix of the mammalian organ or tissue.
Embodiment 39. The method of any one of Embodiments 32 to 38 wherein the composition comprises an agent that reduces the formation of adhesions.
Embodiment 40. The method of any one of Embodiments 32 to 39 further comprising contacting the exterior surface with a composition comprising an agent that reduces the formation of adhesions.
Embodiment 41. The method of Embodiment 39 or 40 wherein the agent comprises polypropylene.
Embodiment 42. The method of Embodiment 39 or 40 wherein the agent comprises laminin, basement membrane substrates or collagen IV.
Embodiment 43. The method of any one of Embodiments 32 to 42 wherein the composition is sprayed onto the exterior surface.
Embodiment 44. The method of any one of Embodiments 32 to 43 wherein the organ or tissue is submerged in the composition.
Embodiment 45. The method of any one of Embodiments 32 to 44 wherein the contacting results in crosslinking of molecules on the exterior surface.
Embodiment 46. The method of any one of Embodiments 32 to 45 wherein the agent is an enzyme.
Embodiment 47. The method of Embodiment 46 wherein the enzyme comprises a transferase, hydrolase or oxidoreductase.
Embodiment 48. The method of Embodiment 46 wherein the enzyme comprises transglutaminase.
Embodiment 49. The method of Embodiment 45 wherein the molecule on the exterior surface that is crosslinked to the at least one agent comprises collagen.
Embodiment 50. The method of any one of Embodiments 32 to 49 wherein the composition comprises a photocrosslinker.
Embodiment 51. The method of any one of Embodiments 32 to 49 wherein the exterior surface is further contacted with a photocrosslinker.
Embodiment 52. The method of Embodiment 50 or 51 wherein the photocrosslinker comprises a polymer.
Embodiment 53. The method of Embodiment 50 or 51 wherein the exterior surface is contacted with a polyvinylpyrrolidone or photopolyvinylpyrrolidone.
Embodiment 54. The method of Embodiment 50 or 51 wherein the exterior surface is contacted with a poly(ethylene), poly(ethylene oxide), poly(vinyl alcohol), copolymers of maleic anhydride and/or phthalic anhydride with styrene and some polymers derived from cinnamic acid.
Embodiment 55. The method of Embodiment 50 or 51 wherein the exterior surface is contacted with comprises benzophenone.
Embodiment 56. The method of any one of Embodiments 32 to 55 wherein the composition comprises one or more amino acids.
Embodiment 57. The method of any one of Embodiments 32 to 56 wherein the composition comprises Type I Collagen, Type 2 Collagen, Type 3 Collagen, Laminin, RGD, or Type 4 collagen.
Embodiment 58. The method of an y one of Embodiments 32 to 57 wherein the at least one agent comprises a bifunctional crosslinker.
Embodiment 59. The method of any one of Embodiments 32 to 58 wherein the composition comprises gelatin, agar or carrageenan.
Embodiment 60. The method of any one of Embodiments 32 to 58 wherein the composition comprises collagen.
Embodiment 61. A product produced by the method of any one of Embodiments 32 to 60.
Embodiment 62. The method of any one of Embodiments 6 to 8 further comprising contacting the exterior surface of the recellularized organ or tissue, or portion thereof, with a composition comprising at least one agent in an amount that forms a biocompatible coating on the exterior surface of the recellularized extracellular matrix of the mammalian organ or tissue, or portion thereof.
Embodiment 63. The method of any one of Embodiments 32 to 60 further comprising decellularizing the coated organ or tissue, or portion thereof.
Embodiment 64. The method of Embodiment 63 further comprising contacting the exterior surface of the decellularized organ or tissue, or portion thereof, with a composition comprising at least one agent in an amount that forms a biocompatible coating on the exterior surface of the decellularized extracellular matrix of the mammalian organ or tissue, or portion thereof.
Embodiment 65. A method to decrease porosity of an outer fibrous layer of a decellularized mammalian organ or tissue, or a portion thereof, comprising:
   contacting an exterior surface of a decellularized extracellular matrix of a mammalian organ or tissue, or portion thereof, which comprises a decellularized extracellular matrix vascular tree, wherein said decellularized extracellular matrix of said organ or tissue, or portion thereof, retains a majority but not all of fluid introduced thereto, with a composition comprising at least one agent in an amount that forms a biocompatible coating on the exterior surface of the decellularized extracellular matrix of the mammalian organ or tissue, or portion thereof, effective to increase retention of fluid introduced to the decellularized extracellular matrix vascular tree relative to a corresponding decellularized extracellular matrix of the mammalian organ or tissue, or portion thereof, comprising an exterior surface comprising decellularized extracellular matrix and a decellularized extracellular matrix vascular tree which is not contacted with the composition.
Embodiment 66. The method of Embodiment 65 further comprising recellularizing the coated decellularized extracellular matrix of the mammalian organ or tissue, or portion thereof.
Embodiment 67. The method of Embodiment 66 further comprising contacting the exterior surface of the recellularized coated extracellular matrix of the mammalian organ or tissue, or portion thereof, with a composition comprising at least one agent in an amount that forms a biocompatible coating on the exterior surface of the recellularized extracellular matrix of the mammalian organ or tissue, or portion thereof.
Embodiment 68. A method to decrease porosity of an exterior surface of a mammalian organ or tissue, or a portion thereof, comprising:
   contacting an exterior surface of a mammalian organ or tissue, or a portion thereof, wherein said organ or tissue, or portion thereof, comprises a vascular tree and cells, with a composition comprising at least one agent in an amount that forms a coating on the exterior surface effective to increase retention of fluid in the organ or tissue relative to fluid introduced to a corresponding organ or tissue, or a portion thereof, prior to exposure to the composition.
Embodiment 69. The method of Embodiment 68 further comprising decellularizing the coated decellularized extracellular matrix of the mammalian organ or tissue, or portion thereof.
Embodiment 70. The method of Embodiment 69 further comprising contacting the exterior surface of the decellularized extracellular matrix of the mammalian organ or tissue, or portion thereof, with a composition comprising at least one agent in an amount that forms a biocompatible coating on the exterior surface of the decellularized extracellular matrix of the mammalian organ or tissue, or portion thereof.

## Claims

1. A method to decrease porosity of an external surface of a mammalian organ or tissue, or a portion thereof, comprising:
providing a mammalian organ or tissue, or portion thereof, which comprises the exterior surface and a vascular tree; and
contacting the exterior surface with a composition comprising at least one photocrosslinker that forms a biocompatible coating on the exterior surface of the mammalian organ or tissue, or portion thereof effective to increase retention of fluid introduced to the organ or tissue relative to a corresponding mammalian organ or tissue, or portion thereof, comprising an exterior surface and a vascular tree which is not contacted with the composition.

2. The method of claim 1, wherein the mammal is a swine, bovine, ovine or human.

3. The method of claim 1 or claim 2, wherein the organ is a liver, heart, spleen, pancreas, bladder, kidney, small bowel, large bowel, stomach, bone, brain, or a lung.

4. The method of any one of claims 1-3, further comprising exposing the composition to ultraviolet (UV) light following contacting the exterior surface in order to initiate crosslinking of the photocrosslinker

5. The method of any one of claims 1-4, wherein the amount of the composition enhances mechanical flexibility or strength of the decellularized extracellular matrix of the mammalian organ or tissue or reduces the formation of adhesions.

6. The method of any one of claims 1-5, further comprising introducing a population of cells to a duct, cavity or one or more vessels of the organ or tissue.

7. The method of any one of claims 1-6, wherein contacting the exterior surface with the composition reduces the formation of adhesions.

8. The method of any one of claims 1-7, wherein the photocrosslinker comprises polyvinylpyrrolidone (PVP), polyethylene glycol (PEG), polypropylene, poly(ethylene), poly(ethylene oxide), poly(vinyl alcohol), copolymers of maleic anhydride or phthalic anhydride with styrene, and/or a polymer derived from cinnamic acid.

9. The method of any one of claims 1-8, wherein the composition further comprises laminin, basement membrane substrates or collagen IV.

10. The method of any one of claims 1-9 wherein the composition is sprayed onto, brushed onto or applied to the exterior surface or the organ or tissue is submerged in the composition.

11. The method of any one of claims 1-10 wherein the composition further comprises Type I Collagen, Type 2 Collagen, Type 3 Collagen, Laminin, arginylglycylaspartic acid (RGD), or Type 4 collagen.

12. The method of any one of claims 1-11, wherein the composition further comprises gelatin, agar, carrageenan, or collagen.

13. The method of any one of claims 1-12, wherein the mammalian organ or tissue, or a portion thereof has not been decellularized or recellularized

14. The method of any one of claims 1-12, wherein the mammalian organ or tissue, or a portion thereof is a decellularized mammalian organ or tissue, or a portion thereof.

15. The method of any one of claims 1-12, wherein the mammalian organ or tissue, or a portion thereof is a recellularized mammalian organ or tissue, or a portion thereof.

16. The method of any one of claim 1-12, further comprising decellularizing the mammalian organ or tissue, or a portion thereof following the contacting.

17. The method of claim 16, further comprising recellularizing the mammalian organ or tissue, or a portion thereof following the decellularizing.

18. The method of claim 17, wherein the contacting is effective to increase retention of fluid introduced to the mammalian organ or tissue, or a portion thereof following the decellularization or recellularization of the mammalian organ or tissue, or a portion thereof relative to a corresponding decellularized or recellularized mammalian organ or tissue, or portion thereof that comprises an exterior surface and a vascular tree which is not contacted with the composition.
